# EUROPÄISCHE PATENTSCHRIFT

(11) **EP 2 219 569 B1**
(45) Veröffentlichungstag und Bekanntmachung des Hinweises auf die Patenterteilung: **20.07.2011**
(21) Anmeldenummer: 08868039.2
(22) Anmeldetag: 09.12.2008
(51) Int. Cl.: A61F 7/08

(54) **Wabenartige Wärmflasche**
Honeycomb-like hot-water bottle
Bouillotte en nid d'abeilles

(30) Priorität: 21.12.2007 DE 202007018248 U
(43) Veröffentlichungstag der Anmeldung: 25.08.2010
(73) Patentinhaber: Fashy GmbH Produktion und Vertrieb Gummi-Kraus, 70825 Korntal-Münchingen (DE)
(72) Erfinder: KRAUS, Alexander, 71229 Leonberg (DE)
(74) Vertreter: Fuhlendorf, Jörn
(86) Internationale Anmeldenummer: PCT/EP2008/010420
(87) Internationale Veröffentlichungsnummer: WO 2009/083106

(56) Entgegenhaltungen:
- CH-A- 281 705
- DE-B- 1 033 850
- DE-C- 225 407
- DE-C- 522 308
- DE-C- 562 327
- FR-A- 1 452 888
- US-A- 1 372 893

## Beschreibung

Die vorliegende Erfindung bezieht sich auf eine wabenartige Wärmflasche nach dem Oberbegriff des Anspruchs 1.

CH 281705 A offenbart eine Wärmflasche nach dem Oberbegriff des Anspruchs 1.

Übliche Wärmflaschen bestehen aus zwei im Spritzgießverfahren hergestellte ungeformte Hälften, die randseitig miteinander verschweißt werden. Diese Wärmflaschen werden beim Befüllen mit beispielsweise warmem Wasser bauchig, da sie in ihrer Form instabil sind.

Deshalb wurden wabenartige Wärmflaschen gemäß dem Oberbegriff des Anspruchs 1 vorgeschlagen, die auch nach dem Befüllen in ihrer Form stabil bleiben sollen. Es hat sich jedoch herausgestellt, dass dies in nicht in allen Punkten zufriedenstellender Weise erreicht worden ist.

Aufgabe der vorliegenden Erfindung ist es deshalb, eine wabenartige Wärmflasche der eingangs genannten Art zu schaffen, die nach dem Befüllen mit beispielsweise warmem Wasser formhaltig bleibt, bei der also beispielsweise die Halbschalen sowohl in ihrer Fläche als auch längs ihrer Ränder in der vorgegebenen Form bleiben.

Zur Lösung dieser Aufgabe sind bei einer wabenartigen Wärmflasche der genannten Art die im Anspruch 1 angegebenen Merkmale vorgesehen.

Durch die erfindungsgemäßen Maßnahmen ist eine Formhaltigkeit auch der gefüllten Wärmflasche erreicht, wobei die inneren angeformten Stege den über einen bestimmten Radius hochgestellten Rand nahezu umlaufend aussteifen und dadurch deren Formhaltigkeit gewährleisten. Herstellungstechnisch ist dies eine einfache Lösung, da die Stege beim Herstellen jeder der Halbschalen mit angeformt werden können.

Vorteilhafte Ausgestaltungen hierzu ergeben sich aus den Merkmalen eines oder mehrerer der Ansprüche 1 bis 5. Dabei ist von besonderem Vorteil, wenn die Stege sich bis in einen flächigen ebenen Bereich der Halbschalen erstrecken. Erfindungsgemäß enden die Stege vor der Randschweißnaht, um diesen Schweißvorgang nicht zu behindern.

Mit den Merkmalen nach Anspruch 6 ist die Formhaltigkeit der ebenen Fläche der beiden Wärmflaschen-Halbschalen erreicht.

Vorteilhafte Ausgestaltungen hinsichtlich der Form der beiden Wärmflaschen-Halbschalen ergeben sich aus den Merkmalen des Anspruchs 7 oder denen des Anspruchs 8.

Mit den Merkmalen des Anspruchs 9 ist ein bevorzugtes Material gewählt, das die bisherigen Materialien, wie Latex oder Gummi, ersetzt und das gemäß Anspruch 10 bevorzugt transparent ist.

Eine besonders günstige Ausgestaltung ergibt sich dann, wenn die Merkmale nach Anspruch 11 verwirklicht sind.

Weitere Einzelheiten der Erfindung sind der folgenden Beschreibung zu entnehmen, in der die Erfindung anhand des in der Zeichnung dargestellten Ausführungsbeispieles näher beschrieben und erläutert ist. Es zeigen:
- Figur 1: in Vorderansicht eine wabenartige Wärmflasche gemäß einem bevorzugten Ausführungsbeispiel vorliegender Erfindung und
- Figur 2: einen Schnitt längs der Linie II-II der Figur 1.

Die in der Zeichnung dargestellte wabenartige Wärmflasche 10 ist aus einem transparenten oder opaken PVC derart hergestellt, dass sie auch nach Befüllen mit beispielsweise warmem Wasser ihre Form behält. Die Wärmflasche 10 ist somit in sich formstabil beziehungsweise formhaltig, d.h. auch, dass sie nicht ausbaucht. Es versteht sich, dass die Wärmflasche 10 auch aus anderen verspritzbaren Materialien hergestellt werden kann.

Die Wärmflasche 10 besteht aus zwei hier identischen Halbschalen 11 und 12, die in einem Spritzgießwerkzeug hergestellt sind und die umfangsseitig randseitig miteinander verbunden, vorzugsweise verschweißt sind. Die Wärmflasche 10 besitzt ferner einen üblichen hier ebenfalls transparenten Füllstutzen 13, der mit einer trichterförmigen Öffnuhg 14 und einem hier nicht dargestellten Schraubverschluss versehen ist. Der Füllstutzen 13 ist mit den beiden Halbschalen 11 und 12 verbunden, vorzugsweise einstückig verspritzt.

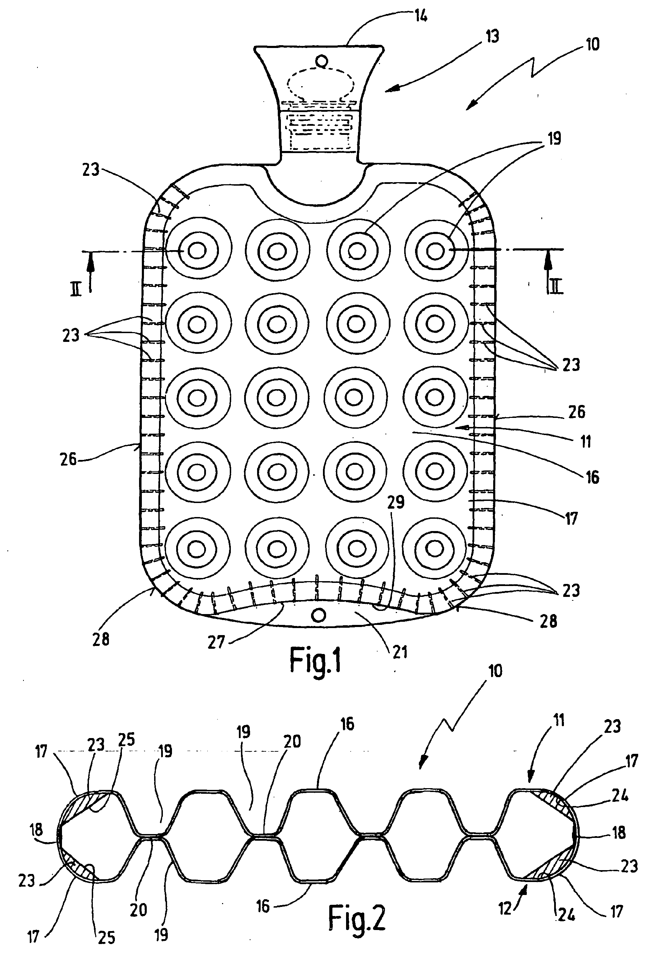

Die beiden Halbschalen 11 und 12 besitzen eine ebene Außenoberfläche 16 und einen über einen Radius hochgebogenen beziehungsweise hochstehenden Rand 17. Der hochstehende Rand 17 ist umlaufend vorgesehen. In Ansicht gesehen besitzt die Wärmflasche 10 eine übliche Außenform, während sie gemäß dem Schnitt nach Figur 2 eine länglich rechteckige längsrandseitig abgerundete Form aufweist, bei der die beiden ebenen Außenoberflächen 16 der Halbschalen 11 und 12 in einem bestimmten Abstand zueinander parallel verlaufen.

Von den ebenen Außenoberflächen 16 jeder Halbschale 11 und 12 gehen über sie gleichmäßig verteilt angeordnete eingeformte Noppen 19 aus, die trichterförmig von außen nach innen verlaufen und einen Boden 20 besitzen. Die Noppen 19 der Halbschalen 11 und 12 sind unmittelbar gegenüberliegend angeordnet und an ihrem Boden 20 jeweils gegenüberliegend miteinander verbunden, vorzugsweise verschweißt.

Die hochstehenden Ränder 17 beider Halbschalen 11 und 12 sind umlaufend entweder miteinander verschweißt oder mit dem Füllstutzen 13 verspritzt. Dem Füllstutzen 13 abgewandt besitzt die Wärmflasche 10 einen Aufhängelappen 21, der einen konkaven Außenumfangsbereich 29 ausfüllt und am betreffenden Bereich der Schweißnaht 18 ansetzt.

Im Bereich des hochgestellten Randes 17 der beiden Halbschalen 11 und 12 sind Versteifungsstege 23 vorgesehen, die von der Innenfläche 24 des Randes 17 ausgehen und an dieser angeformt sind. Die Versteifungsstege 23 besitzen eine geradlinig verlaufende freie Stirnfläche 25 zur Innenseite der Halbschale 11, 12 hin. Die Versteifungsstege 23 sind etwa 1 bis 2 mm dick. Sie verlaufen von einem ebenen Bereich der Halbschale 11, 12 aus über den bogenförmig hochgezogenen Rand 17 und enden vor der Schweißnaht 18. Die Versteifungsstege 23 sind bis auf den Verbindungsbereich der Halbschalen 11, 12 mit dem Füllstutzen 13 über den gesamten Umfang der Wärmflasche 10 beziehungsweise deren Halbschalen 11 und 12 gleichmäßig verteilt angeordnet. Im Längsrandbereich 26 der beiden Halbschalen 11, 12 sind die Versteifungsstege 23 in gleichmäßigem Abstand parallel zueinander angeordnet, während sie in den gebogenen Querrandbereichen 27 und den gebogenen Übergangsbereichen 28 der Halbschalen 11, 12 in gleichmäßigem Abstand strahlenförmig verlaufen.

Wie erwähnt, werden die Halbschalen 11 und 12 zusammen mit dem Füllstutzen 13 und einschließlich der Noppen 19 und Versteifungsstege 23 in einem Werkzeug im Spritzgussverfahren hergestellt. Die Außenfläche der Halbschale 11 und/oder 12 kann dabei eine erhabene Oberlächenstruktur, wie Lamellen, Rippen, Prägungen o. dgl. erhalten. Danach werden die beiden mit dem Füllstutzen 13 versehenen Halbschalen 11 und 12 in ein weiteres Werkzeug eingelegt und längs der Randstirnflächen und an den einander zugewandten mit ihrem Boden 20 anliegenden Noppen 19 miteinander verschweißt.

## Patentansprüche

1. Wabenartige Wärmflasche (10) aus zwei randseitig verschweißten Halbschalen (11, 12) und einem daran angespritzten verschließbaren Füllstutzen (13), wobei die Wärmflaschen-Halbschalen (11, 12) mit einem umlaufenden und über einen Radius hochgestellten Rand (17) und mit über die Innenfläche vorzugsweise gleichmäßig verteilt angeordneten von außen nach innen eingeformten Noppen (19) versehen sind, **dadurch gekennzeichnet, dass** jede der beiden mittels Spritzguss hergestellten Wärmflaschen-Halbschalen (11, 12) im Bereich ihres Randes (17) mit von dessen Innenfläche (24) vorstehenden angeformten Stegen (23) versehen ist und dass die Stege (23) vor der Randschweißnaht (18) der beiden Wärmflaschen-Halbschalen (11, 12) enden.

2. Wärmflasche nach Anspruch 1, **dadurch gekennzeichnet, dass** die Stege (23) der Innenfläche (24) der Wärmflaschen-Halbschale (11, 12) abgewandt eine geradlinig verlaufende freie Stirnfläche (25) besitzen.

3. Wärmflasche nach Anspruch 1 oder 2, **dadurch gekennzeichnet, dass** die Stege (23) bis in einen zum Rand (17) benachbarten ebenen Bereich der Wärmflaschen-Halbschale (11, 12) verlaufen.

4. Wärmflasche nach mindestens einem der vorhergehenden Ansprüche, **dadurch gekennzeichnet, dass** die Stege (23) über einen wesentlichen Teil des Umfanges der Wärmflaschen-Halbschalen (11, 12) gleichmäßig verteilt angeordnet sind.

5. Wärmflasche nach mindestens einem der vorhergehenden Ansprüche, **dadurch gekennzeichnet, dass** die Stege (23) im Bereich der geraden Längsseitenränder (26) der Wärmflaschen-Halbschalen (11, 12) parallel und im Bereich der gebogenen Seitenränder (27, 28) der Wärmflaschen-Halbschalen (11, 12) strahlenartig verlaufen.

6. Wärmflasche nach mindestens einem der vorhergehenden Ansprüche, **dadurch gekennzeichnet, dass** die Noppen (19) der beiden Wärmflaschen-Halbschalen (11, 12) gegenüberliegend miteinander verschweißt sind.

7. Wärmflasche nach mindestens einem der Ansprüche 1 bis 6, **dadurch gekennzeichnet, dass** beide Wärmflaschen-Halbschalen (11, 12) eine ebene bzw. glatte Außenoberfläche besitzen.

8. Wärmflasche nach mindestens einem der Ansprüche 1 bis 6, **dadurch gekennzeichnet, dass** die eine und/oder andere Wärmflaschen-Halbschale (11, 12) eine mit einer erhabenen Oberflächenstruktur versehene Außenoberfläche besitzt bzw. besitzen.

9. Wärmflasche nach mindestens einem der vorhergehenden Ansprüche, **dadurch gekennzeichnet, dass** die Wärmflaschen-Halbschalen (11, 12) aus PVC gespritzt sind.

10. Wärmflasche nach mindestens einem der vorhergehenden Ansprüche, **dadurch gekennzeichnet, dass** sie aus transparentem PVC ist.

11. Wärmflasche nach mindestens einem der vorhergehenden Ansprüche, **dadurch gekennzeichnet, dass** sie so ausgebildet ist, dass sie beim Befüllen nicht ausbaucht.

## Claims

1. Honeycomb-like hot water bottle (10) comprising two half shells (11, 12) welded at the edges thereof and a closable filler neck (13) which is injection moulded thereon, wherein the hot water bottle half shells (11, 12) are provided with a peripheral edge (17) which is raised over a radius, and with knobs (19) formed inwardly from the outside, distributed, preferably evenly, over the interior surface, **characterised in that** each of the hot water bottle half shells (11, 12) formed by injection moulding is provided in the region of the edge (17) thereof with moulded webs (23) projecting from the interior surface (24) of said half shell and that the webs (23) end before the edge weld seam (18) of the two hot water bottle half shells (11, 12) .

2. Hot water bottle according to claim 1, **characterised in that** the webs (23) of the interior surface (24) of the hot water bottle half shells (11, 12) have a free end face (25) extending straight and facing away from the interior surface (24) of the hot water bottle half shell (11, 12).

3. Hot water bottle according to claim 1 or 2, **characterised in that** the webs (23) extend as far as a planar region of the hot water bottle half shell (11, 12) adjacent to the edge (17).

4. Hot water bottle according to at least one of the preceding claims, **characterised in that** the webs (23) are arranged evenly distributed over a substantial part of the periphery of the hot water bottle half shells (11, 12).

5. Hot water bottle according to one of the preceding claims, **characterised in that** the webs (23) extend parallel in the region of the straight longitudinal side edges (26) of the hot water bottle half shells (11, 12) and extend radially in the region of the curved side edges (27, 28) of the hot water bottle half shells (11, 12).

6. Hot water bottle according to at least one of the preceding claims, **characterised in that** the knobs (19) of the two hot water bottle half shells (11, 12) are welded to one another in opposing manner.

7. Hot water bottle according to at least one of the claims 1 to 6, **characterised in that** both hot water bottle half shells (11, 12) have a planar or smooth exterior surface.

8. Hot water bottle according to at least one of the claims 1 to 6, **characterised in that** one and/or the other hot water bottle half shell (11, 12) has/have an exterior surface provided with a raised surface structure.

9. Hot water bottle according to at least one of the preceding claims, **characterised in that** the hot water bottle half shells (11, 12) are injection moulded from PVC.

10. Hot water bottle according to at least one of the preceding claims, **characterised in that** said hot water bottle is made from transparent PVC.

11. Hot water bottle according to one of the preceding claims, **characterised in that** said hot water bottle is configured so as not to bulge during filling.

## Revendications

1. Bouillotte alvéolée (10) formée de deux demi-enveloppes (11, 12) soudées périphériquement et d'une tubulure de remplissage obturable (13) solidaire desdites demi-enveloppes et obtenue par injection, les demi-enveloppes de bouillotte (11, 12) présentant un bord (17) périphérique relevé selon un rayon de courbure et des bossages (19) rentrants, de préférence uniformément répartis sur la surface interne, **caractérisée en ce que** chacune des deux demi-enveloppes de bouillotte (11, 12) moulées par injection présente, dans la région de son bord (17), des éléments de renfort (23) formés en saillie sur sa surface interne (24) et **en ce que** lesdits éléments de renfort (23) se terminent avant le cordon de soudure périphérique (18) des deux demi-enveloppes de bouillotte (11, 12).

2. Bouillotte selon la revendication 1, **caractérisée en ce que** les éléments de renfort (23) présentent, à l'opposé de la surface interne (24) des demi-enveloppes de bouillotte (11, 12), une face frontale (25) libre rectiligne.

3. Bouillotte selon la revendication 1 ou la revendication 2, **caractérisée en ce que** les éléments de renfort (23) s'étendent jusqu'à une zone plane des demi-enveloppes de bouillotte (11, 12) adjacente au bord (17).

4. Bouillotte selon l'une au moins des revendications précédentes, **caractérisée en ce que** les éléments de renfort (23) sont uniformément répartis sur l'essentiel de la périphérie des demi-enveloppes de bouillotte (11, 12).

5. Bouillotte selon l'une au moins des revendications précédentes, **caractérisée en ce que** les éléments de renfort (23) sont disposés parallèlement au niveau des bords droits longitudinaux (26) des demi-enveloppes de bouillotte (11, 12) et sont disposés radialement au niveau des bords incurvés (27, 28) des demi-enveloppes de bouillotte (11, 12).

6. Bouillotte selon l'une au moins des revendications précédentes, **caractérisée en ce que** les bossages (19) en regard les uns des autres des deux demi-enveloppes de bouillotte (11, 12) sont soudés entre eux.

7. Bouillotte selon l'une au moins des revendications 1 à 6, **caractérisée en ce que** les deux demi-enveloppes de bouillotte (11, 12) présentent une surface externe plane ou lisse.

8. Bouillotte selon l'une au moins des revendications 1 à 6, **caractérisée en ce que** l'une et/ou l'autre des demi-enveloppes de bouillotte (11, 12) présente(nt) une surface externe dotée d'une surface reliéfée.

9. Bouillotte selon l'une au moins des revendications précédentes, **caractérisée en ce que** les demi-enveloppes de bouillotte (11, 12) sont injectées en PVC.

10. Bouillotte selon l'une au moins des revendications précédentes, **caractérisée en ce qu'**elle est en PVC transparent.

11. Bouillotte selon l'une au moins des revendications précédentes, **caractérisée en ce qu'**elle est conformée de manière à ne pas prendre une forme ballonnée lors du remplissage.
